Europäisches Patentamt

European Patent Office

Office européen des brevets

(19)

(11) Veröffentlichungsnummer: **0 057 359**
**B1**

(12) # EUROPÄISCHE PATENTSCHRIFT

(45) Veröffentlichungstag der Patentschrift:
04.01.84

(51) Int. Cl.³: **C 12 N 9/64**

(21) Anmeldenummer: **82100283.9**

(22) Anmeldetag: **16.01.82**

(54) Verfahren zur Herstellung von Kallikrein aus Schweinepankreas-Extrakten.

(30) Priorität: **31.01.81 DE 3103257**

(43) Veröffentlichungstag der Anmeldung:
**11.08.82 Patentblatt 82/32**

(45) Bekanntmachung des Hinweises auf die Patenterteilung:
**04.01.84 Patentblatt 84/1**

(84) Benannte Vertragsstaaten:
**DE FR IT**

(73) Patentinhaber: **BAYER AG, Zentralbereich Patente, Marken und Lizenzen, D-5090 Leverkusen 1, Bayerwerk (DE)**

(72) Erfinder: **Schutt, Hermann, Dr., Gellertweg 12, D-5600 Wuppertal 1 (DE)**

(56) Entgegenhaltungen:
FR - A - 2 210 620
FR - A - 2 283 696

THE JOURNAL OF IMMUNOLOGY, Band 121, Nr. 1, Juli 1978, Seiten 66-71, Baltimore USA ONESMO OLE-MOIYOI et al.: "Inhibition of human urinary kallikrein (urokallikrein) by anti-enzyme fab"
CHEMICAL ABSTRACTS, Band 93, Nr. 14, 6. Oktober 1980, Seiten 342, 343, Nr. 137977p, Columbus, Ohio, USA PHARMACIA FINE CHEMICALS, "Ion exchange chromatography" März 1980, Uppsala 1, SW
JOURNAL OF CHROMATOGRAPHY, Band 210, Nr. 1, Mai 1981, Seiten 67-76, Amsterdam, NL. B.A. KLYASCHCHITSKY et al.: "Use of biospecific adsorbents with polysaccharide spacers in affinity chromatography"

Verfahren zur Herstellung von Kallikrein aus Schweinepankreas-Extrakten

Die vorliegende Erfindung betrifft ein Verfahren zur Herstellung von Kallikrein aus Organextrakten, insbesondere aus Schweinepankreas-Extrakten, wobei die proteolytische Begleitaktivität des Schweinepankreas-Extraktes durch Chromatographie an Anionenaustauschern abgetrennt und das hochreine Enzym Kallikrein durch Chromatographie an Trägermaterialien mit einem in spezieller Weise kovalent gebundenen Kallikrein-Trypsin-Inhibitor (basic pancreatic trypsin inhibitor = BPTI) in hochreiner Form erhalten wird.

Das Enzym Kallikrein wurde mit seinen biochemischen Eigenschaften von F. Fiedler, Methods in Enzymology, Vol. 45, Proteolytic Enzmyes, Part B, 289–303, beschrieben.

Bei Einwirkung auf körpereigenes Kininogen setzt Kallikrein die physiologisch-wirksamen Kinine (z.B. Kallidin) frei. Kallikrein-Präparate werden daher therapeutisch verwendet [E.K. Frey, H. Kraut, E. Werle, Das Kallikrein-Kinin-System und seine Inhibitoren, F. Enke, Stuttgart (1968), 150 ff.].

Das Enzym Kallikrein wurde bereits in einheitlicher Form aus vorgereinigten Extrakten des Schweinepankreas durch Chromatographie an Ionenaustochersäulen erhalten [C. Kutzbach und G. Schmidt-Kastner, Hoppe-Seyler's Z. Physiol. Chem. 353 (1972), 1099–1106, DE-A 2 154 556 und DE-A 2 154 557).

Die genannten Verfahren sind jedoch unspezifisch und technisch aufwendig, da ausgehend vom Organextrakt insgesamt 5 Schritte zur Gewinnung des reinen Enzyms Kallikrein notwendig sind. Die auf der biospezifischen Wechselwirkung von Inhibitor und Enzym bestehende Technik der Affinitätschromatographie kommt in der Theorie mit einem Isolierungsschritt aus und wird daher in letzter Zeit bevorzugt zur Enzymisolierung im technischen Massstab angestrebt.

Für Kallikrein wurden bereits verschiedene Verfahren zur Affinitätschromatographie in der Literatur bekannt, die auf der Wechselwirkung von trägergebundenem BPTI mit Kallikrein beruhen.

BPTI bildet mit Kallikrein in Abhängigkeit des angewandten pH-Wertes reversibel einen Enzym-Inhibitor-Komplex [H. Tschesche, Angewandte Chemie 86 (1974), 24–40; E. Auhagen, International Symposium on Drugs of Animal Origin, A. Leonardi und J. Walsh ed., Ferro edizioni Mailand S. 69].

Der BPTI ist aufgrund seiner pH-, thermischen, proteolytischen und Lösungsmittel-Stabilität [B. Kassell, Methods in Enzymology 19 (1970), 844–852] verglichen mit anderen polyvalenten Inhibitoren, wie z.B. dem Soya-Bohnen-Trypsin-Inhibitor, besonders zur Isolierung der proteolytischen Enzyme aus Organextrakten geeignet. Durch die Bindung an den BPTI ist das proteolytische Enzym weitgehend vor einer Inaktivierung geschützt.

Der BPTI ist ein aus Rinderorganen gewonnenes Polypeptid mit 58 Aminosäuren bekannter Primärstruktur [B. Kassell und M. Laskowski, Biochem. Biophys. Res. Commun. 20 (1965), 463–468 und F.A. Anderer und S. Hörnle, J. Biol. Chem. 241 (1966), 1568–1572].

Der BPTI wurde nach D.A. Johnson und J. Travis, Anal. Biochem. 72 (1976), 573–576, G.J. Baugh und J. Travis, Biochemistry 15 (1976), 836–841; R. Geiger, W. Stuckstedte und H. Fritz, Hoppe-Seyler's Z. Physiol. Chem. 361 (1980), 1003–1016 sowie nach der DE-OS 2 363 201 an mit Bromcyan aktivierte hydroxylgruppenhaltige Träger kovalent gebunden und zur Affinitätschromatographie von proteolytischen Enzymen eingesetzt.

Ferner ist die kovalente Bindung des BPTI an Anhydridharze nach DE-A 1 768 934 und H. Fritz, B. Brey, A. Schmal und E. Werle, Hoppe-Seyler's Z. Physiol. Chem. 350 (1969), 617–625 sowie die kovalente Bindung des BPTI mittels wasserlöslicher Carbodiimide an Carboxylgruppen tragende Sepharose 4 B nach G.R. Geiger, R. Mann und T. Bettels, J. Clin. Chem. Biochem. 15 (1977), 479–483, O. Ole-Moi Yoi, J. Spragg und K.F. Austen, J. Immunol. 121 (1978), 66–71, N.B. Oza und R.W. Ryan, Biochem. J. 171 (1978), 285–288, N.B. Oza, V.M. Amin, R.K. McGregor, A.G. Scidi und D.A. Carretero, Biochem. Pharmacol. 25 (1976), 1607–1612, zum Zwecke der Affinitätschromatographie bekanntgeworden.

Die bekannt gewordenen Verfahren sind aber mit wesentlichen Nachteilen behaftet, die ihren wirtschaftlichen Einsatz stark beschränken.

So ist die affinitätschromatographische Reinigung von Enzymen nicht allein von der biospezifischen Wechselwirkung des Effektors (Inhibitors) mit dem zu isolierenden Enzym abhängig, sondern die Wirtschaftlichkeit des Verfahrens hängt wesentlich mit von der verwendeten Kupplungsmethode für den Effektor und der chemischen Struktur des polymeren Trägers ab.

So ist. z.B. bekanntgeworden, dass die nach Aktivierung hydroxylgruppenhaltiger Träger mit Bromcyan entstehende Isoharnstoffbindung kationisch geladen und instabil ist [G.I. Tesser, H.U. Fisch und R. Schwyzer, Helvetica chimica acta 57 (1974), 1718–1730].

Nach der DE-A 1 768 934 resultiert ein anionischer BPTI-Träger, der zur Isolierung von Kallikrein mit Diaminen neutralisiert werden muss. Nach beiden Verfahrensweisen wird kein stabiler BPTI-Träger und kein reines Kallikrein erhalten. Nachteilig und unwirtschaftlich ist auch das Vorgehen der DE-A 2 363 201. Ein Schweinepankreas-Extrakt wird ohne Vorreinigung auf einer BPTI-Sepharose 4 B-Säule zu Trypsin, Chymotrypsin und Kallikrein aufgearbeitet. Trypsin und Chymotrypsin sind wesentlich billiger als Kallikrein. Zur Kallikrein-Isolierung aus Trypsin-haltigem Schweinepankreas-Extrakt wird jedoch der grösste Anteil der äusserst teuren BPTI-Sepharose 4 B zur Isolierung des billigen Trypsins verwendet, da einmal die Konzentration des Trypsins und Chymotrypsins in Schweinepankreas etwa 100mal grösser als die des Kallikreins ist [C. Kutzbach und G. Schmidt-Kastner, Kininogenases F.K. Schattauer-Verlag, Stuttgart – New York (1973), 23–25] und zum anderen der Trypsin-BPTI-Komplex [für Rinder-Trypsin: $K_i = 6,0 \times 10^{-14}$ M bei pH 8,0; M. Lazdunski, J.B. Vincent, H. Schweitz, M. Peron-Renner, J. Pudlers (1974) in Proteinase Inhibitors – Bayer Symposium V; H. Fritz, H. Tschesche, L.J. Greene, E. Truscheit eds. 420–431, Springer-Verlag, Berlin] wesentlich weniger dissoziiert ist als der Kallikrein-BPTI-Komplex [für Schweinepankreas-Kallikrein, $K_i = 1 \times 10^{-9}$ M bei pH 8,0 H. Fritz, H. Schult, R. Meister und E. Werle, Hoppe-Seyler's Z. Physiol. Chem. 350 (1969), 1531–1540].

Es wurde ein verbessertes Verfahren zur Herstellung von Kallikrein gefunden, das dadurch gekennzeichnet ist, dass ein Kallikrein enthaltender Organextrakt auf eine Leitfähigkeit von 2 bis 6 mScm$^{-1}$ und einen pH-Wert von 5 bis 8 eingestellt wird, der so vorbereitete Organextrakt mit einem Ionenaustauscher in Batch- oder Säulenform in Kontakt gebracht und das Kallikrein durch kontinuierliche oder diskontinuierliche Erhöhung der Ionenstärke mittels eines ein- oder zweiwertigen Alkali- oder Erdalkalisalzes oder durch Senkung des pH-Wertes eluiert wird, das Eluat durch Affinitätschromatographie an kovalent trägergebundenem BPTI nachgereinigt und bei einem pH-Wert von 4,0 bis 5,0 von BPTI-Träger desorbiert wird.

Nach dem beschriebenen Verfahren gelangt man in technisch einfacher und wirtschaftlicher Weise vom Organextrakt ausgehend mit 0,5-Kallikrein-Einh./mg in zwei Schritten zu reinem Kallikrein mit 1000–1300 KE/mg. [Die Kallikrein-Aktivität wird durch Messung der Hydrolyse von N-Benzoyl-L-argininethyl-ester in der von der F.I.P. (Fédération Internationale Pharmaceutique) standardisierten Ausführungsform als titrimetrischer Test bestimmt. 1 F.I.P.-Einheit ist definiert als die Kallikreinmenge, die in einer Minute bei pH 8,0 und 25°C 1 Mikromol N-Benzoyl-L-argininethylester spaltet. Die Umrechnung in die gebräuchlichen Kallikrein-Einheiten (KE) nach der Definition von Frey, Kraut und Werle erfolgt durch Multiplikation mit dem Faktor 6,37].

Das Verfahren gliedert sich einmal in einen Vorreinigungsschritt an Anionenaustauschern. Zu diesem Zweck muss der Schweinepankreas-Organextrakt auf eine Leitfähigkeit von 2–6 mS × cm$^{-1}$ und einen pH-Wert von 5–8 eingestellt werden. Die vorbereitete Roh-Kallikrein-Lösung wird mit dem Ionenaustauscher in Batch- oder Säulenform in Kontakt gebracht und Kallikrein durch Erhöhung der Ionenstärke mittels eines ein- oder zweiwertigen Alkali- oder Erdalkalisalzes vom Trägermaterial in gereinigter Form eluiert.

Die Erhöhung der Ionenstärke kann in kontinuierlicher oder diskontinuierlicher Form als Gradient oder in Stufenform erfolgen. Die Elution von Kallikrein vom Ionenaustauscher ist alternativ durch Senkung des pH-Wertes möglich.

Die Verwendung von basischen Ionenaustauschern auf Cellulosebasis zur Reinigung von Kallikrein ist grundsätzlich in der US-A 3 100 736 beschrieben.

Ionenaustauscher auf Cellulosebasis haben die Nachteile der mikrobiologischen Abbaubarkeit und geringer physikalischer Stabilität. Dies schränkt ihre grosstechnische Verwendung ein.

Zur Kallikrein-Reinigung wurden daher auch Anionenaustauscher auf der Basis basisch substituierter makroporöser Polymerisate nach der DE-A 2 424 118, US-A 3 809 748 und US-A 4 038 141 beschrieben. Verglichen mit den Ionenaustauschern auf Cellulosebasis ist ihre Kapazität sowie ihr Reinigungsfaktor für Kallikrein begrenzt.

Die physikalischen Eigenschaften der Ionenaustauscher auf Cellulose-, Sephadex- oder Sepharosebasis lassen sich durch Behandlung mit einem bifunktionellen Reagenz, wie z.B. Epichlorhydrin oder 2,3-Dibrompropanol, nach US-A 3 573 277 erheblich verbessern. Die durch kovalente cross-links modifizierten Ionenaustauscher haben erheblich höhere Durchflussraten als die nativen Ionenaustauscher und können in Säulenform zur Kallikrein-Reinigung verwendet werden.

Die Behandlung der genannten Anionenaustauscher mit den bifunktionellen Reagenzien kann vor oder nach Einführung der ionischen zum Austausch befähigten Gruppen erfolgen, wobei die Behandlung mit dem quervernetzenden Reagenz vor der Einführung der Ionenaustauscher-Gruppen bevorzugt ist.

Die für die beschriebene Kallikrein-Reinigung verwendeten Anionenaustauscher können entweder durch Behandlung von DEAE-Celluose, DEAE-Sephadex® (Dextran der Firma Pharmacia AB, Uppsala, Schweden) oder DEAE Sepharose® (Agarose der Firma Pharmacia AB, Uppsala, Schweden) mit bifunktionellen Reagenzien hergestellt oder als quervernetzte Anionenaustauscher, wie z.B. DEAE-Sephacel® (ein mit Epichlorhydrin quervernetzter Celluloseaustauscher der Firma Pharmacia AB, Uppsala, Schweden) oder DEAE-Indion®-Ionenaustauscher (ein mit Epichlorhydrin quervernetzter Celluloseaustauscher der Firma Phoenix Chemicals Ltd., Waitaki, Neuseeland) käuflich erworben werden.

Wegen ihrer grossen Kapazität für Kallikrein können die quervernetzten Ionenaustauscher auch zur weiteren Verbesserung der Durchflussrate mit inerten Trägergelen, wie z.B. Sephadex® G 10, G 15 oder G 25 der Firma Pharmacia AB, Uppsala, Schweden, in verschiedenen Verhältnissen gemischt in Säulenform eingesetzt werden.

Ausgehend von Schweinepankreas-Organextrakt mit einer Aktivität von 0,5–1 KE/mg kann Kallikrein durch Behandlung mit den genannten Ionenaustauschern auf eine spezifische Aktivität von 15–120 KE/mg angereichert werden. Die proteolytische Aktivität des Schweinepankreas-Extrakts in Form der proteolytischen Enzyme Trypsin und Chymotrypsin wird weitgehend abgetrennt.

Die weitere Reinigung der Kallikrein-Zwischenstufe zu Kallikrein erfolgt an einem speziellen BPTI-Träger. Vom Organextrakt ausgehend wird für Kallikrein insgesamt ein Reinigungsfaktor von 1000–2500fach erzielt. Das gewonnene Kallikrein hat eine spezifische Aktivität von 1000–1350 KE/mg.

Als polymere Trägermaterialien für die kovalente Bindung des BPTI können hydroxylgruppenhaltige Polymere, wie z.B. Sephadex® (quervernetztes unlösliches Dextran der Firma Pharmacia AB, Uppsala, Schweden), Sepharose® (unlösliche Agarose = lineares Polysaccharid aus D-Galactose und 3,6-Anhydro-L-Galactose der Firma Pharmacia AB, Uppsala, Schweden) sowie Dextran oder Cellulose verwendet werden.

Um einen in Säulenform verwendbaren BPTI-Träger zu erhalten, wird der polymere Träger mit einer grossen Menge des zur kovalenten Bindung des BPTI verwendeten bifunktionellen Reagenzes behandelt, um durch Einfügen von kovalenten cross-links das Trägermaterial zu festigen.

Die verwendeten bifunktionellen Reagenzien sind entweder symmetrisch oder unsymmetrisch aufgebaut (F. Wold, Methods in Enzymology 11, S. 617 ff.). Sie sind chemisch bevorzugt den Halohydrinen, Bisalkylhalogeniden, bifunktionellen Epoxiden oder den Divinylverbindungen zuzuordnen. Stellvertretend jedoch nicht einschränkend seien folgende Reagenzien genannt: Epichlorhydrin, Epibromhydrin, Dibrompropanol, 1,4-Butandioldiglycidylether [L. Sundberg und J. Porath, J. Chrom. 90 (1974), 87–98; Bisoxiran und Divinylsulfon (DE-OS 2 312 615)].

Die kovalente Bindung des BPTI an den polymeren Träger ist in allen Fällen nichtionisch und sehr stabil. Die BPTI-Träger können mindestens 20mal zur Kallikrein-Reinigung ohne Verlust der Kallikrein-Kapazität eingesetzt werden.

Die zur Bindung des BPTI verwendeten bifunktionellen Reagenzien führen gleichzeitig mit der Bindung des BPTI zu einer Quervernetzung. Die daraus resultierende verbesserte Durchflussgeschwindigkeit und bessere mikrobielle Stabilität des Trägermaterials ist technisch durchaus erwünscht.

Überraschenderweise kann die vom Anionenaustauscher erhaltene vorgereinigte Kallikrein-Fraktion, die bis zu 1 M Salz enthalten kann, ohne Entzalzung auf die BPTI-Säule aufgetragen werden. Der optimale pH-Wert für die Kallikrein-Bindung liegt bei pH 6–8. Durch Nachwaschen mit 0,2 M Natriumacetatpuffer pH 6–5 können Verunreinigungen ausgewaschen werden. Kallikrein wird unterhalb von pH 5,0 vom BPTI-Träger eluiert, wobei der pH-Wert wegen der pH-Stabilität von Kallikrein 4,0 nicht unterschreiten sollte.

Überraschenderweise werden mit dem erhaltenen BPTI-Träger über 99,9% der noch in der vorgereinigten Kallikrein-Fraktion in Form von Chymotrypsin und anderer Proteasen vorhandenen proteolytischen Aktivität abgetrennt. Dies war nicht vorhersehbar, da die Gleichgewichtskonstanten des BPTI-Kallikrein-Komplexes und des BPTI-Chymotrypsin-Komplexes mit $6\times10^{-9}$ M bei pH 7,2 für Chymotropysin [B. Bösterling und J. Engel, Hoppe-Seyler's Z. Physiol. Chem. 357 (1976), 1297–1307] und mit $1\times10^{-9}$ M bei pH 8,0 für Kallikrein [H. Fritz, H. Schult, R. Meister und E. Werle, Hoppe-Seyler's Z. Physiol. Chem. 350 (1969), 1531–1540] nahezu gleich sind.

Die vollständige Abtrennung von proteolytischer Aktivität aus Kallikrein-Präparaten ist umso wichtiger als nach der DE-A 2 442 995 ein direkter Zusammenhang zwischen Protease-Gehalt der Kallikrein-Präparate und der Haltbarkeit von Kallikrein in Lösung besteht.

Die Abtrennung der Proteasen erfolgt ansonsten in einem speziellen Schritt mittels trägergebundener aus Sojabohne oder Kartoffel isolierter Inhibitoren, die Kallikrein nicht zu hemmen vermögen.

Beispiele

1. Beispiele zur Vorreinigung des Schweinepankreas-Organextraktes mit Anionenaustauschern

Beispiel 1: Ausrührung von Schweinepankreas-Organextrakt im Batch mit nativer oder modifizierter DEAE-Cellulose

a) Herstellung des Organextraktes: 3,5 kg tiefgefrorener Schweinepankreas wird in einem Fleischwolf zerkleinert. Zu dem Organbrei werden 7 l Leitungswasser zugefügt und der pH-Wert mit Essigsäure auf pH 4,8 eingestellt. Zur Entfettung werden 3,5 l ≙ 5 kg Trichlorethylen zugegeben und eine Stunde bei 25°C gerührt. Es werden 1 kg Filterhilfsmittel zugefügt und die Suspension 15 Minuten gerührt. Das Bindegewebe wird abgeschöpft und Reste über eine mit Kieselgur belegte Filterpresse entfernt. Die Trichlorethylen enthaltende untere Schicht wird abgetrennt, die wässrige Phase mit 0,2 kg Kieselgur versetzt und die Lösung über Seitz AS-Filterplatten klarfiltriert. Kallikrein-Ausbeute: $0,22\times10^6$ KE.

b) Herstellung von modifizierter DEAE-Cellulose (Cl⁻-Form): 45 g DEAE-Cellulose (Typ 130, Schleicher und Schüll GmbH, Dassel/BRD) werden in 150 ml 1 N NaOH suspendiert und 2 Stunden bei 60°C unter Rühren nach Zugabe von 25 ml Epichlorhydrin quervernetzt. Die modifizierte DEAE-Cellulose wird auf der Nutsche mit dest. Wasser gewaschen und mit HCl in die Chloridform überführt.

c) Adsorption von Kallikrein an DEAE-Cellulose (Cl⁻-Form): 1000 kg Pankreas-Organextrakt hergestellt nach Beispiel 1a) mit einem pH-Wert von 5,0–5,5 und einer Leitfähigkeit von 4,5–6,2 mS × cm⁻¹ werden in einem 3000 l-Kessel ausgerüstet mit einem Ankerrührer durch Zufügen von 1500 l entsalztem Wasser auf eine Leitfähigkeit von 3,0–3,3 mS × cm⁻¹ verdünnt und der pH-Wert durch Zugabe von 1,5–2 l 45%iger NaOH auf pH 7,0 eingestellt. Es werden 15–20 kg feuchte native bzw. nach Beispiel 1b modifizierte DEAE-Cellulose (Chloridform) zugefügt und zur Adsorption von Kallikrein 60 Minuten ausgerührt. Die feuchte DEAE-Cellulose wird mit einer Schlauchpumpe mit 800 l/Std. auf eine Vollmantelschleuder gepumpt und bei 2000–3000 g aus der Suspension abgetrennt. Der Schleudervorlauf wird sofort mit konz. HCl auf pH 1,8 eingestellt und zur Aufarbeitung auf Chymotrypsin/Trypsin aufbewahrt. Die abgetrennte feuchte native oder modifizierte DEAE-Cellulose wird zur Ablösung von Kallikrein nacheinander mit 60, 30 und 10 l 0,05 M Phosphatpuffer pH 5,0 +0,5 M NaCl jeweils 60 Min. bei Raumtemperatur ausgerührt, zwischendurch wird über ein Seitz-Einschichtenfilter (D = 60 cm) filtriert. Die vereinigten Filtrate mit einer Leitfähigkeit von 32–40 mS × cm$^{-1}$ werden unter Rühren auf pH 7,0 eingestellt und können zur Herstellung des reinen Kallikreins direkt auf eine BPTI-Träger-Säule aufgetragen werden.

Daten der Schweinepankreas-Organlösung (jeweils pro 1000 kg):
Proteingehalt = 8,8–15,3 kg
Kallikrein-Aktivität = 28,1×10$^6$ KE.
Trypsin-Aktivität = 6,0–26,8×10$^6$ F.I.P.-Einheiten
Chymotrypsin-Aktivität: 204,5–510,4×10$^6$ ATEE-Einheiten (1 ATEE-Einheit entspricht der Enzymmenge, die 1 Mikromol N-Acetyl-L-tyrosinethylester/Minute hydrolysiert).
Daten der über DEAE-Cellulose vorgereinigten Kallikrein-Lösung:
Proteingehalt: 0,552–1,34 kg
Kallikrein-Aktivität = 20,9×10$^6$ KE = 74,4%
Trypsin-Aktivität: 0,1×10$^6$ F.I.P.-Einheiten
Chymotrpysin-Aktivität: 0,2–4×10$^6$ ATEE-Einheiten.

Beispiel 2: Chromatographie von Schweinepankreas-Organextrakt auf DEAE-Sephacel®
Eine Säule (Höhe = 5,4 cm, Durchmesser = 8,0 cm, Volumen = 271,3 ml) gefüllt mit DEAE-Sephacel® wird mit 0,05 M Phosphatpuffer pH 7,0 äquilibriert. Bei einem Durchfluss von 900 ml/Std. werden 5 l Schweinepankreas-Extrakt nach Beispiel 1a), der auf pH 7,0 und mit Wasser auf eine Leitfähigkeit von 4,1 mS × cm$^{-1}$ eingestellt wurde, aufgetragen. Die Säulenfüllung wird mit 3,8 l 0,05 M Phosphatpuffer pH 7,0 nachgewaschen und die Kallikrein-Aktivität mit einem linearen Gradienten aus 2 l 0,05 M Phosphatpuffer pH 7,0 auf 2 l 0,05 Phosphatpuffer pH 7,0 +0,5 M NaCl eluiert.
Kallikrein-Aktivität wird bei einer Leitfähigkeit von 10–12 mS × cm$^{-1}$ vom Säulenmaterial eluiert. Die aktiven Fraktionen werden vereinigt und durch Dialyse entsalzt.
Ausgangsaktivität = 172.00 KE mit 45,42 g Protein
Kallikrein-Eluat = 125.256 KE mit 1,4 g Protein.

2. Beispiele zur Herstellung der BPTI-Träger

Beispiel 3: Herstellung von Sepharose 4 B®-Epichlorhydrin-BPTI
250 ml sedimentierte Sepharose 4 B® werden in 200 ml 1 N NaOH suspendiert und 2 Stunden mit 25 ml Epichlorhydrin bei 60°C unter Rühren aktiviert. Das aktivierte Gel wird abgesaugt und auf der Nutsche mehrfach mit dest. Wasser gewaschen. Anschliessend wird die aktivierte Sepharose 4 B® in 250 ml 0,01 N NaOH suspendiert, 1,25 g BPTI mit einer Hemmaktivität von 7100 Kallikrein-Einheiten = KIE/mg [Kallikrein-Trypsin-Inhibitor-Einheiten, als Literaturstelle: E.K. Frey, H. Kraut und E. Werle «Das Kallikrein-Kinin-System und seine Inhibitoren», S. 11, Ferdinand Enke Verlag, Stuttgart (1968)] zugefügt und die Suspension über Nacht bei Raumtemperatur gerührt. Der fertige BPTI-Träger wird abgesaugt, auf der Nutsche mehrfach mit dest. Wasser, 10%ige NaCl-Lösung und wieder mit dest. Wasser gewaschen. Das Filtrat enthielt 1,48×10$^6$ KIE entsprechend 16,7% der eingesetzten BPTI-Aktivität.

Beispiel 4: Herstellung von Sepharose 4 B®-Cyanurchlorid-BPTI
500 ml sedimentierte Sepharose 4 B® werden 60 Minuten lang in einer Mischung aus 250 ml dest. Wasser und 500 ml Dioxan bei pH 5,0–7,0 mit 25 g Cyanurchlorid aktiviert. Die aktivierte Sepharose 4 B® wird abgesaugt, auf der Nutsche mit Dioxan und dest. Wasser gewaschen und als Suspension bei pH 7,0–8,0 unter Zusatz von 5 g partiell gereinigtem BPTI (4716 KIE/mg) 16 Stunden bei Raumtemperatur gekuppelt. Der fertige BPTI-Träger wird abgesaugt, auf der Nutsche mehrfach mit dest. Wasser, 10%iger NaCl-Lösung und wieder mit Wasser gewaschen. Das Filtrat enthält 0,25×10$^6$ KIE entsprechend <1,1% der eingesetzten BPTI-Aktivität.

Beispiel 5: Herstellung von Sepharose 4 B®-Bromcyan-BPTI
500 ml sedimentierte Sepharose 4 B® werden 30 Minuten nach Zugabe von 4,1 g Bromcyan bei pH 11,0 unter Zufügen von 1 N NaOH bei konstantem pH-Wert aktiviert.
Der pH-Wert wird der Suspension durch Zugabe von 2 N HCl auf 7,0 eingestellt, 5 g partiell gereinigter BPTI (4716 KIE/mg) zugefügt und 16 Stunden bei konstantem pH und Raumtemperatur gerührt.
Der fertige BPTI-Träger wird abgesaugt, auf der Nutsche mehrfach mit dest. Wasser, 10%iger NaCl-Lösung und wieder mit dest. Wasser gewaschen. Das Filtrat enthält 11,72×10$^6$ KIE entsprechend 49,7% der eingesetzten BPTI-Aktivität.

Beispiel 6: Herstellung von Sepharose 4B®-Divinylsulfon-BPTI
50 ml sedimentierte Sepharose 4 B® werden in 100 ml 0,1 M Na$_2$CO$_3$-Lösung bei pH 11,0 mit 5 ml Divinylsulfon 2 Stunden bei Raumtemperatur gerührt.
Die aktivierte Sepharose 4 B® wird abgesaugt, mit dest. Wasser gewaschen und anschliessend in 50 ml 0,1 M Na$_2$CO$_3$-Lösung bei pH 11,0 mit 1 g BPTI (7100 KIE/mg) über Nacht gerührt.
Die fertige BPTI-Sepharose 4 B® wird abgesaugt, auf der Nutsche mit dest. Wasser, 10%iger NaCl-Lösung und wieder mit dest. Wasser gewaschen. Es werden nach Absaugen 40,27 g feuchte Sepharose 4 B®-Divinylsulfon-BPTI erhalten.

Das Filtrat enthielt $0,036 \times 10^6$ KIE entsprechend 0,6% der eingesetzten BPTI-Aktivität.

3. Beispiele zur Herstellung von reinem Kallikrein mit BPTI-Trägern

Beispiel 7: Herstellung von Rein-Kallikrein mit Sepharose 4 B®-Epichlorhydrin-BPTI

Nach Beispiel 3 hergestellter Sepharose 4 B®-Epichlorhydrin-BPTI-Träger wird in eine Säule (Höhe = 15 cm, Durchmesser = 37 cm, Volumen = 16 l) gefüllt. Das Gelbett wird bei einem Durchfluss von 7 l/Std. mit ca. 20 l 0,1 M Phosphatpuffer pH 7,0 äquilibriert.

25–50 l des nach Beispiel 1c) hergestellten DEAE-Cellulose-Desorbates, mit NaOH eingestellt auf pH 7,0, wird bei einem Durchlauf von 7 l/Std. von unten auf die Säule gepumpt. Bei der gleichen Pumpgeschwindigkeit wird mit 40 l 0,05 M Phosphatpuffer pH 7,0+0,5 M NaCl nachgewaschen. Es folgen beim gleichen Durchfluss ca. 40 l 0,2 M Natriumacetatpuffer pH 5,0–6,5 bis die UV-Absorption den Ausgangswert erreicht hat. Kallikrein wird mit ca. 40 l 0,05–0,3 M Natriumacetatpuffer pH 4,4 eluiert, die sofort mit konz. $NH_3$ (1:1 verd. mit $H_2O$) auf pH 6,0–6,5 eingestellt werden.

Adsorbierte Verunreinigungen werden von dem BPTI-Träger anschliessend mit ca. 40 l 0,1 N HCl desorbiert. Zuletzt wird die Säulenfüllung mit 40–60 l 0,1 M Phosphatpuffer pH 7,0 gespült, bis der pH-Wert im Säulenablauf pH 7,0 anzeigt.

Der neutralisierte Kallikrein-Hauptlauf wird auf einem DDS-0,36 $m^2$-Labormodul, bestückt mit 20 DDS-600 Ultrafiltern, durch Hinzufügen von entsalztem Wasser zum Rezirkulat entsalzt und zuletzt bei einer Leitfähigkeit des Rezirkulates von ca. 1 mS × $cm^{-1}$ und einer Kallikrein-Konzentration von ca. 1000 KE/ml auf 2–3 l konzentriert und gefriergetrocknet.

Das Kallikrein-Eluat kann alternativ durch Dialyse oder Chromatographie auf einer Sephadex G 25-coarse-Säule weiter entsalzt werden.
Eingesetztes DEAE-Cellulose-Desorbat:

46,2 kg Lösung mit 316,0 g Protein
Kallikrein-Aktivität: $9,46 \times 10^6$ KE
Proteolytische Aktivität: 632.000 Kunitz-Einheiten
(Der Proteasengehalt wird gemessen durch Titration der freigesetzten Amino-Carboxylgruppen bei der Einwirkung der proteasehaltigen Kallikrein-Lösung auf Casein. Man verwendet eine 6%ige Lösung von Casein (nach Hammersten, Merck 2242) in 0,1 N KCl und titriert mit 0,02 N KOH bei pH 9,5 und 30°C. 1 Proteasen-Einheit ist definiert als die Enzymmenge, die in einer Minute unter den angegebenen Bedingungen eine Mikroäquivalent-Peptidbindung spaltet.)
Säulendurchlauf bei pH 7,0 = 84,9 kg mit 305,1 g Protein und einer Kallikrein-Aktivität von $0,35 \times 10^6$ KE $\triangleq$ 3,7% der Ausgangsaktivität.
Nachwaschen des BPTI-Trägers mit 0,2 M Natriumacetat pH 5,0 = 41,3 kg mit 9,70 g Protein und einer Kallikrein-Aktivität von $1,43 \times 10^6$ KE $\triangleq$ 15,1% der Ausgangsaktivität.

Kallikrein-Eluat bei pH 4,4: 39,5 kg Lösung mit 3,48 g Protein und einer Kallikrein-Aktivität von $5,61 \times 10^6$ KE $\triangleq$ 59,3% der eingesetzten Aktivität.
Charakterisierung des erhaltenen Kallikreins:

1. Spezifische Aktivität = 1210 KE/mg
2. Aschegehalt: 0,88%
3. Proteolytische Aktivität = 0,097 Kunitz-Einheiten/mg = 337,6 Kunitz-Einheiten insgesamt = 0,05% der proteolytischen Ausgangs-Aktivität
4. Kininase-Gehalt: <0,5 mg/Min./KE
5. Carboxypeptidase B (Substrat: Hippuryl-Arg) <0,1%
6. Carboxypeptidase A (Substrat: Hippuryl-Phe) <1%
7. UV-Absorption bei 280 nm, $E_{280}^{0,1\%} = 1,78$
UV-Absorption bei 260 nm, $E_{260}^{0,1\%} = 1,07$
E 280/E 260 = 1,66
8. Cellogel-Elektrophorese: Das erhaltene Kallikrein-Präparat besteht aus Kallikrein A und B (Bedingungen s. US-P 3 905 870). Proteine ohne Kallikrein-Aktivität werden nicht beobachtet.

Maximale Kallikrein-Kapazität der Sepharose 4 B®-Epichlorhydrin-BPTI-Säule für die Kallikrein-DEAE-Cellulose-Zwischenstufe: 800.000 KE/l Säulenmaterial.

Beispiel 8: Herstellung von Kallikrein mit Sepharose 4 B®-Divinylsulfon-BPTI

Es wird der in Beispiel 6 hergestellte Sepharose 4 B®-Divinylsulfon-BPTI-Träger eingesetzt. Die übrigen Bedingungen gleichen Beispiel 7 mit der Ausnahme, dass das Nachwaschen der Säulenfüllung bei pH 5,0 vor der Elution des Rein-Kallikreins weggelassen wird.
Eingesetztes DEAE-Cellulose-Desorbat:
25 kg Lösung mit 117,0 g Protein
Kallikrein-Aktivität = $2,72 \times 10^6$ KE
Proteolytische Aktivität: 234.000 Kunitz-Einheiten
Säulenvorlauf bei pH 7,0: 87,5 kg mit 126,0 g Protein und einer Kallikrein-Aktivität von 0 KE
Kallikrein-Eluat bei pH 4,4: 34,1 kg Lösung mit 3,6 g Protein und einer Kallikrein-Aktivität von $2,7 \times 10^6$ KE $\triangleq$ 100% der eingesetzten Kallikrein-Aktivität.
Lyophilisiertes Rein-Kallikrein: 790 KE/mg
Proteolytische Aktivität = 0,745 Kunitz-Einheiten/mg = 2682 Kunitz-Einheiten = 1,1% der proteolytischen Ausgangsaktivität.

4. Vergleichsbeispiele mit anderen BPTI-Trägern

Beispiel 9: Herstellung von Kallikrein mit Sepharose 4 B®-Cyanurchlorid-BPTI

Nach Beispiel 4 hergestellter Sepharose 4 B®-Cyanurchlorid-BPTI-Träger wird in eine Säule (Höhe = 8,3 cm, Durchmesser = 10,6 cm, Volumen = 732,1 ml) gefüllt.

Bei einem Durchfluss von 300 ml/Std. wird ein nach Beispiel 1c) hergestelltes DEAE-Cellulose-Desorbat aufgetragen und wie in Beispiel 7 beschrieben weiter aufgearbeitet.
Ausgangsaktivität: 599.040 KE

Säulendurchlauf bei pH 7,0 = 112.944 KE ≙ 18,8% der Ausgangsaktivität.

Nachwaschen des BPTI-Trägers mit 0,2 M Natriumacetatpuffer pH 5,0 = 122.928 KE = 20,5% der Ausgangsaktivität.

Kallikrein-Eluat bei pH 4,4: 227.430 KE = 38,0% der eingesetzten Kallikrein-Aktivität.

Lyoausbeute = 319,2 mg mit 686% KE/mg und einer proteolytischen Aktivität von 0,796 Kunitz-Einheiten/mg.

Die spezifische Aktivität und die proteolytische Aktivität können durch erneute Chromatographie an dem BPTI-Träger nicht verbessert werden.

In der Cellogelelektrophorese auf Cellogelstreifen 40×170 mm in 0,15 M Phosphatpuffer pH 7,65 wird neben der Kallikrein A- und B-Bande eine schwächer saure Proteinbande ohne Kallikrein-Aktivität beobachtet.

Beispiel 10: Herstellung von Kallikrein nach DOS 2 363 210 mit Sepharose 4 B®-Bromcyan-BPTI

Nach Beispiel 5 hergestellter Sepharose 4 B®-Bromcyan-BPTI-Träger wird in eine Säule (Höhe = 4,1 cm, Durchmesser = 3,6 cm, Volumen = 41,7 ml) gefüllt.

Es werden 750 ml nach Beispiel 1a) hergestellter Organextrakt aufgetragen und mit 0,05 M Phosphatpuffer pH 7,0 nachgewaschen. Verunreinigungen werden mit 0,2 M Natriumacetatpuffer pH 5,0 eluiert. Kallikrein wird mit 0,3 M Natriumacetatpuffer pH 4,4 eluiert. Trypsin- und Chymotrypsin-Aktivität werden mit 0,1 N HCl vom BPTI-Träger eluiert.

Aktivitätsbilanz für Kallikrein:
Ausgangsaktivität = 5700 KE
Säulenvorläufe = 0 KE
Kallikrein-Eluat = 3753 KE ≙ 65,8% der Ausgangsaktivität

Kallikrein-Kapazität des Sepharose 4 B®-Bromcyan-BPTI-Trägers für Schweinepankreas-Extrakt: 136.690 KE/L BPTI-Träger. Spezifische Aktivität des Kallikrein-Lyopulvers: 17,7 KE/mg. In der Cellogelelektrophorese auf Cellogelstreifen 40×170 mm in 0,5 M Phosphatpuffer pH 7,65 werden neben der Kallikrein A- und B-Bande 4 weitere schwächer saure Proteinbanden ohne Kallikrein-Aktivität beobachtet.

Aktivitätsbilanz für Trypsin:
Ausgangsaktivität: 2025 F.I.P.-Einheiten
Säulenvorläufe = 0 F.I.P.-Einheiten
Trypsin-Eluat = 608 F.I.P.-Einheiten ≙ 30,0% der Ausgangsaktivität.

Aktivitätsbilanz für Chymotrypsin:
Ausgangsaktivität = 244.500 ATEE-Einheiten
Säulenvorläufe = 204.844 ATEE-Einheiten ≙ 83,8% der Ausgangsaktivität
Chymotrypsin-Eluat = 0 ATEE-Einheiten.

Das erhaltene Trypsin und Chymotrypsin sind nach der Elektrophorese und spezifischen Aktivität beurteilt als unrein zu bezeichnen.

## Patentansprüche

1. Verfahren zur Herstellung von Kallikrein, dadurch gekennzeichnet, dass ein Kallikrein enthaltender Organextrakt auf eine Leitfähigkeit von 2 bis 6 mS cm$^{-1}$ und einen pH-Wert von 5 bis 8 eingestellt wird, der so vorbereitete Organextrakt mit einem Ionenaustauscher in Batch- oder Säulenform in Kontakt gebracht und das Kallikrein durch kontinuierliche oder diskontinuierliche Erhöhung der Ionenstärke mittels eines ein- oder zweiwertigen Alkali- oder Erdalkalisalzes oder durch Senkung des pH-Wertes eluiert wird, das Eluat durch Affinitätschromatographie an kovalent trägergebundenem BPTI nachgereinigt und bei einem pH-Wert von 4,0 bis 5,0 vom BPTI-Träger desorbiert wird.

2. Verfahren nach Anspruch 1, dadurch gekennzeichnet, dass als Ionenaustauscher und BPTI-Träger solche auf Cellulose-, Dextran- oder Agarosebasis eingesetzt werden, die durch Behandlung mit bifunktionellen Reagenzien quervernetzt worden sind.

3. Verfahren nach Anspruch 2, dadurch gekennzeichnet, dass die Quervernetzung des BPTI an den Träger mit bifunktionellem Reagenz erfolgt.

4. Verfahren nach einem oder mehreren der Ansprüche 1 bis 3, dadurch gekennzeichnet, dass der Organextrakt mindestens 0,5 KE/mg enthält.

5. Verfahren nach einem oder mehreren der Ansprüche 1 bis 4, dadurch gekennzeichnet, dass die Erhöhung der Ionenstärke als Gradient oder in Stufenform vorgenommen wird.

6. Verfahren nach Anspruch 1, dadurch gekennzeichnet, dass die Behandlung mit dem quervernetzenden Reagenz vor der Einführung der Ionenaustauscher-Gruppen vorgenommen wird.

7. Verfahren nach einem oder mehreren der Ansprüche 1 bis 6, dadurch gekennzeichnet, dass die quervernetzten Ionenaustauscher mit inerten Trägergelen vermischt werden.

8. Verfahren nach einem oder mehreren der Ansprüche 1 bis 7, dadurch gekennzeichnet, dass die bifunktionellen Reagenzien chemisch den Halohydrinen, Bisalkylhalogeniden, bifunktionellen Epoxiden oder Divinylverbindungen zuzurechnen sind.

9. Verfahren nach einem oder mehreren der Ansprüche 1 bis 8, dadurch gekennzeichnet, dass die bifunktionellen Reagenzien eines oder mehrere aus der Gruppe: Epichlorhydrin, Epibromhydrin, Dibrompropanol, 1,4-Butandioldiglycidylether, Bisoxiran und Divinylsulfon sind.

10. Verfahren nach einem oder mehreren der Ansprüche 1 bis 9, dadurch gekennzeichnet, dass die von Anionenaustauscher vorgereinigte Fraktion ohne zwischengeschaltete Entsalzung auf die BPTI-Säule aufgetragen wird.

## Claims

1. Process for the preparation of kallikrein, characterised in that an organ extract containing kallikrein is adjusted to a conductivity of 2 to 6 mS cm$^{-1}$ and a pH value of 5 o 8, the organ extract thus prepared is brought into contact with an ion exchanger in batch form or column form and the kallikrein is eluted by continuously or discontinuously increasing the ionic strength by means of a mono- or divalent alkali metal salt or alkaline earth metal salt or by lowering the pH value, the eluate is subsequently purified by affinity chromatography on BPTI covalently bonded to a carrier and is desorbed from the BPTI-carrier at a pH value of 4.0 to 5.0.

2. Process according to Claim 1, characterised in that the ion exchanger and BPTI-carrier used are based on cellulose, dextran or agarose and have been crosslinked by treatment with bifunctional reagents.

3. Process according to Claim 2, characterised in that the crosslinking of the BPTI on the carrier takes place using a bifunctional reagent.

4. Process according to one or more of Claims 1 to 3, characterised in that the organ extract contains at least 0.5 KU/mg.

5. Process according to one or more of Claims 1 to 4, characterised in that the ionic strength is increased in gradient form or stepwise.

6. Process according to Claim 1, characterised in that the treatment with the crosslinked reagent takes place before the introduction of the ion exchanger groups.

7. Process according to one or more of Claims 1 to 6, characterised in that the crosslinked ion exchangers are mixed with inert carrier gels.

8. Process according to one or more of Claims 1 to 7, characterised in that the bifunctional reagents belong, from a chemical point of view, to the halohydrins, bisalkyl halides, bifunctional epoxides or divinyl compounds.

9. Process according to one or more of Claims 1 to 8, characterised in that the bifunctional reagents are one or more from the group comprising epichlorohydrin, epibromohydrin, dibromopropanol, 1,4-butanediol diglycidyl ether, bisoxirane and divinyl sulphone.

10. Process according to one or more of Claims 1 to 9, characterised in that the fraction pre-purified by the anion exchanger is discharged on to the BPTI column without intermediate demineralisations.

## Revendications

1. Procédé de production de kallicréine, caractérisé en ce qu'on ajuste un extrait d'organe contenant de la kallicréine à une conductivité de 2 à 6 mS cm$^{-1}$ et à un pH de 5 à 8, on met l'extrait d'organe ainsi préparé en contact avec un échangeur ionique par lots ou sur colonne et on élue la kallicréine par élévation continue ou discontinue de la force ionique au moyen d'un sel monovalent ou divalent de métal alcalin ou alcalino-terreux ou par abaissement de la valeur du pH, on purifie ensuite l'éluat par chromatographie par affinité sur un BPTI lié par covalence à un support et on effectue la désorption du support de BPTI à une valeur de pH de 4,0 à 5,0.

2. Procédé suivant la revendication 1, caractérisé en ce qu'on utilise comme échangeur d'ions et comme support de PBTI des produits à base de cellulose, de dextrane ou d'agarose qui ont été réticulés par traitement avec des réactifs difonctionnels.

3. Procédé suivant la revendication 2, caractérisé en ce que la réticulation du BPTI sur le support est effectuée avec un réactif difonctionnel.

4. Procédé suivant une ou plusieurs des revendications 1 à 3, caractérisé en ce que l'extrait d'organe contient au moins 0,5 unité de kallicréine par milligramme.

5. Procédé suivant une ou plusieurs des revendications 1 à 4, caractérisé en ce que l'élévation de la force ionique est effectuée selon un gradient ou par paliers.

6. Procédé suivant la revendication 1, caractérisé en ce que le traitement avec l'agent de réticulation est effectué avant l'introduction des groupes d'échange ionique.

7. Procédé suivant une ou plusieurs des revendications 1 à 6, caractérisé en ce que les échangeurs ioniques réticulés sont mélangés avec des gels inertes de support.

8. Procédé suivant une ou plusieurs des revendications 1 à 7, caractérisé en ce que les réactifs bifonctionnels comptent, du point de vue chimique, parmi les halogénhydrines, des bishalogénures d'alkyle, des époxydes bifonctionnels ou des composés divinyliques.

9. Procédé suivant une ou plusieurs des revendications 1 à 8, caractérisé en ce que les réactifs bifonctionnels consistent en un ou plusieurs réactifs du groupe suivant: épichlorhydrine, épibromhydrine, dibromopropanol, éther de glycidyle du 1,4-butanediol, bisoxirane, et divinylsulfone.

10. Procédé suivant une ou plusieurs des revendications 1 à 9, caractérisé en ce que la fraction préalablement débarrassée de l'échangeur anionique est chargée sans élimination intermédiaire des sels sur la colonne de BPTI.